# FASCICULE DE BREVET EUROPEEN

(11) **EP 3 341 114 B1**
(45) Date de publication et mention de la délivrance du brevet: **19.06.2019**
(21) Numéro de dépôt: 16758082.8
(22) Date de dépôt: 02.08.2016
(51) Int. Cl.: B01J 8/36, B01J 8/00

(54) **DISPOSITIF D'INJECTION DE FLUIDES DANS LA ZONE LIBRE D'UN LIT FLUIDIFIÉ ROTATIF**
VORRICHTUNG ZUM EINSPRITZEN VON FLÜSSIGKEITEN IN DIE FREIFLÄCHE EINER ROTIERENDEN WIRBELSCHICHT
DEVICE FOR INJECTING FLUIDS INTO THE FREE AREA OF A ROTATING FLUIDISED BED

(30) Priorité: 24.08.2015 BE 201500206
(43) Date de publication de la demande: 04.07.2018
(73) Titulaire: De Broqueville, Axel, 1390 Grez-Doiceau (BE)
(72) Inventeur: De Broqueville, Axel, 1390 Grez-Doiceau (BE)
(74) Mandataire: De Clercq & Partners
(86) Numéro de dépôt international: PCT/BE2016/000036
(87) Numéro de publication internationale: WO 2017/031556

(56) Documents cités:
- EP-A1- 1 958 699
- BE-A3- 1 020 683
- DE-A1-102008 056 952
- US-B2- 8 257 657

## Description

### Domaine technique de l'invention

La présente invention se rapporte aux dispositifs permettant d'améliorer la séparation des fluides et des particules solides formant un lit fluidifié rotatif tournant à l'intérieur d'une chambre cyclonique fixe et aux procédés de transformations des fluides et des solides utilisant ces dispositifs.

### Arrière plan technologique de l'invention

L'utilisation de la force centrifuge pour séparer les particules solides des fluides dans des séparateurs cycloniques ou cyclones est bien connue. Afin de réduire l'entraînement des particules solides le long des parois fixes, vers la sortie centrale des fluides, le brevet DE 10 2008 056 952 A1 décrit un dispositif d'injection d'air autour du tube central d'évacuation des fluides afin de former un anneau rotatif écartant les particules solides du tube central et la demande de brevet européen EP 1 958 699 A1 décrit un dispositif d'injection de fluide le long de la paroi circulaire pour améliorer la ségrégation des particules. Ces dispositifs n'éliminent pas l'entraînement des particules solides venant du côté opposé au tube central le long d'une zone autour de l'axe de symétrie, là où la force centrifuge est très faible. Pour y remédier, le séparateur cyclonique est généralement vertical et le rapport entre la longueur ou hauteur et le diamètre du séparateur cyclonique est grand, de préférence supérieur à 3. En outre, le dispositif d'injection d'air secondaire tel qu'il est décrit dans le brevet DE 10 2008 056 952 A1 peut générer des reflux turbulents entraînant les particules solides vers le tube central.

La formation de lits fluidifiés rotatifs dans une chambre cyclonique fixe ou chambre "vortex", est connue. La force centrifuge du tourbillon libre tournant dans la zone libre, entourée par le lit fluidifié rotatif, augmente plus vite que l'inverse du carré du rayon, ce qui permet de séparer les particules solides des fluides plus légers. Toutefois, l'obtention de lits fluidifiés rotatifs denses et stables avec des particules fines ou poudres nécessitent des chambres cycloniques étroites, dont la largeur est de préférence inférieure à son rayon, afin d'éviter la formation de chenaux par où les fluides peuvent s'échapper en n'ayant que très peu de contact avec les particules solides. Cette étroitesse augmente l'influence des surfaces fixes en contact avec ledit tourbillon libre. La friction à l'intérieur du tube central et le long des parois latérales de la chambre cyclonique fixe ralentit la vitesse de rotation des fluides, favorisant les pertes de particules solides qui longent les parois latérales où la vitesse de rotation est plus faible et la vitesse radiale est plus grande. En outre, l'obtention de lits fluidifiés rotatifs denses et stables avec des poudres nécessitent des débits de fluides très élevés et une force centrifuge très grande, de préférence des centaines de fois supérieure à la gravité, ce qui nécessite une dépense importante d'énergie et génère de l'attrition des poudres.

Des dispositifs rotatifs mécaniques, comme un disque rotatif décrit dans le brevet BE 1020683 ou une cheminée centrale rotative, décrites dans le brevet US 8257657 au nom du même inventeur permettent d'améliorer la séparation des fluides et des particules d'un lit fluidifié rotatif. Ces solutions ont les inconvénients liés aux dispositifs mécaniques tournant à grande vitesse à l'intérieur de chambres cycloniques.

La présente invention permet d'accélérer la vitesse de rotation des fluides dans la zone libre du lit fluidifié rotatif, principalement le long des parois latérales, et donc d'éviter l'entraînement des particules solides le long desdites parois latérales, sans nécessiter de grandes vitesses de rotation du lit fluidifié rotatif, tout en évitant les contraintes mécaniques liées à l'utilisation d'objets tournant rapidement dans des chambres cycloniques. Elle permet également d'obtenir des anneaux rotatifs de fluides suffisamment uniformes pour générer peu de turbulences nuisibles. Elle peut aussi contribuer à l'amélioration de procédés de transformations de fluides et/ou de solides utilisant les chambres cycloniques.

### Sommaire de l'invention

La présente invention est un dispositif d'injection de fluides secondaires à l'intérieur de la zone libre d'un lit fluidifié rotatif dans une chambre cyclonique fixe, comprenant une paroi périphérique (4) et deux parois latérales (3) et (5) ; un dispositif d'alimentation de fluide (57) au travers d'ouvertures (72) réparties le long de ladite paroi périphérique, dans une direction principalement tangentielle (73); un dispositif d'alimentation et d'évacuation de particules solides, (59) et (63), généralement entraînées par un fluide, au travers desdites parois latérales ou de ladite paroi périphérique ; au moins un tube central (7) d'évacuation des fluides tournant dans ladite chambre cyclonique au travers d'une dite paroi latérale; caractérisé en ce qu'il comprend un dispositif d'alimentation de fluides secondaires (17) et (17.1) au travers de chambres d'alimentation centrale (15) et (15.1), situées le long de chacune desdites parois latérales, ayant des ouvertures d'injection (18) et (18.1), réparties autour de l'axe de symétrie cylindrique (1), permettant d'injecter lesdits fluides secondaires, dans une direction principalement tangentielle (19) et (19.1), afin de former deux anneaux rotatifs (44) et (44.1) de fluides secondaires tournant autour de l'axe de symétrie cylindrique (1), le long desdites parois latérales, à l'intérieur de ladite zone libre, et en ce que lesdits dispositifs d'alimentation desdits fluides secondaires permettent d'alimenter lesdits fluides secondaires à une pression suffisante pour faire tourner lesdits anneaux rotatifs de fluides secondaires à une vitesse supérieure et de préférence au moins une fois et demi supérieure à la plus grande vitesse de rotation desdits fluides tournant dans ladite zone libre, lorsque celle-ci est en fonctionnement sans le dispositif d'injection de fluide secondaire selon l'invention.

Afin de former un lit fluidifié rotatif stable et dense, la distance moyenne entre les deux dites parois latérales est généralement inférieure au diamètre moyen et de préférence inférieure au rayon moyen de ladite paroi périphérique et la distance entre les ouvertures (72) d'alimentation de fluide (59) au travers de ladite paroi périphérique est généralement inférieure et de préférence inférieure à la moitié de son rayon moyen.

Suivant un mode particulier de l'invention, ladite chambre d'alimentation centrale (15.1) d'un fluide secondaire (17.1) fixée à ladite paroi latérale (5), opposée audit tube central (7), entoure un autre tube central (7.1) d'évacuation des fluides.

Suivant un autre mode particulier de l'invention, ladite chambre cyclonique, comprenant au moins undit tube central d'évacuation des fluides traversant chacune desdites parois latérales, est divisée en deux sections transversales, A et B, par une paroi de séparation (53), pourvue d'au moins un passage (39) le long de ladite paroi périphérique et dont la partie centrale comprend au moins une chambre d'alimentation centrale (15.3) et (15.4) reliées à un ou plusieurs tubes (13.2) et (13.3) permettant d'alimenter un ou plusieurs fluides secondaires (17.2) et (17.3) injectés au travers d'ouvertures d'injection (18.2) et (18.3) dans une direction principalement tangentielle (19.2) et (19.3) autour de l'axe de symétrie cylindrique (1), le long des deux côtés de ladite paroi de séparation (53), dans lesdites zones libres desdites sections transversales.

Suivant un mode préféré de ce mode particulier lesdits passages (39) sont répartis alternativement de manière à coïncider avec l'amont d'une dite ouverture (72) d'une section transversale et l'aval d'une dite ouverture (72) de l'autre section transversale. De cette manière, la différence de pression statique entre l'amont et l'aval accentue le transfert des particules solides d'une section transversale à l'autre.

La dimension et le nombre desdits passages (39), permettant le passage des particules solides entraînées par des fluides d'une dite section transversale à l'autre et les dimensions desdites deux sections transversales A et B sont à déterminer en fonction des besoins du procédé qui utilise ledit dispositif.

L'injection à grande vitesse desdits fluides secondaires par lesdites ouvertures d'injection génère des variations périodiques de pressions statiques et dynamiques et donc de la turbulence, qui est transmise aux fluides tournant dans la chambre cyclonique. Cette turbulence est généralement indésirable, car elle peut faciliter l'entraînement des particules solides vers le tube central. C'est pourquoi, suivant un mode préféré de l'invention, le nombre desdites ouvertures d'injection souhaité est d'au moins 8 et de préférence au moins 12 pour au moins une et de préférence pour chacune desdites chambres d'alimentation centrale. Suivant ce mode préféré de l'invention, au moins un dit et de préférence chacun desdits anneaux rotatifs de fluides secondaires est formé derrière des parois de guidage, de (38) à (38.3) qui peuvent être les parois latérales (3) ou (5) ou aussi un disque rotatif (50), afin d'améliorer leur homogénéité avant d'entrer en contact avec les fluides tournant dans la chambre cyclonique.

Lorsque le dispositif selon l'invention est en fonctionnement, lesdits anneaux rotatifs de fluides secondaires tournant à l'intérieur de la chambre cyclonique plus rapidement que les fluides avoisinants, sont poussés par la force centrifuge le long desdites parois latérales et de ladite paroi de séparation vers ladite paroi périphérique, formant ainsi un reflux de fluides secondaires, de (35) à (35.3) générant des tourbillons, appelés ci-après tourbillons toroïdaux secondaires, de (36) à (36.3), tournant à la fois autour de l'axe de symétrie (1) et autour de zones circulaires (27) à l'intérieur des sections axiales desdits tourbillons toroïdaux secondaires.

Lesdits reflux de fluides secondaires qui tournent autour de l'axe de symétrie (1) plus rapidement que les flux de fluides sortants, de (30) à (30.3), qui longent lesdites parois latérales et de séparation en entraînant des particules solides, écartent lesdits flux de fluides sortants desdites parois latérales et de séparation et accélèrent leur vitesse de rotation. Si la vitesse de rotation desdits reflux de fluides secondaires est suffisamment élevée, la plus grande force centrifuge refoule vers ladite paroi périphérique une partie, dite partie refoulée, de (33) à (33.3) desdits flux de fluides sortants avec la plupart des particules solides entraînées par lesdits flux de fluides sortants, formant ou renforçant ainsi d'autres tourbillons de type toroïdal (32) tournant à la fois autour de l'axe de symétrie de la chambre cyclonique et de zones circulaires (27) à l'intérieur des sections axiales desdits tourbillons de type toroïdal. L'autre partie, dite partie évacuée, de (34) à (34.3), desdits flux de fluides sortants, déchargée de la plupart des particules solides, contourne lesdits tourbillons toroïdaux secondaires. Une partie, dite partie recyclée (9) et (9.1) est recyclée avec les fluides secondaires et l'autre partie, dite fluides sortant centralement (6) est évacuée centralement par le ou lesdits tubes centraux.

La force de refoulement desdits reflux de fluides secondaires, qui est proportionnelle au carré des vitesses de rotation desdits anneaux rotatifs de fluides secondaires, est également proportionnelle au débit desdits fluides secondaires qui doit être suffisamment élevé, pour obtenir un refoulement significatif, car le transfert de moment cinétique de rotation devient inefficient lorsque les différences de vitesses sont très grandes. Il est donc souhaitable d'avoir un débit desdits fluides secondaires suffisamment élevé, dans chacune desdites chambres d'alimentation centrale, d'au moins 5% et de préférence au moins 8% du débit des fluides traversant la chambre cyclonique.

Suivant un mode particulier de l'invention, ladite paroi latérale (5), opposée audit tube central (7) est équipée d'un disque rotatif latéral (50) permettant de guider ledit anneau rotatif de fluide secondaire (17.1) en tournant autour de l'axe de symétrie de la chambre cyclonique, à proximité de ladite paroi latérale (5), actionné par un dispositif de rotation qui permet de le faire tourner à une vitesse supérieure à la vitesse de rotation des fluides tournant dans ladite zone libre, afin d'augmenter la force de refoulement du reflux de fluide secondaire (35.1) générant le tourbillon toroïdal secondaire (36.1). Les dimensions dudit disque rotatif et sa vitesse de rotation peuvent être déterminées en fonction des besoins du procédé qui utilise la chambre cyclonique.

La vitesse de rotation des fluides sortant centralement (6) est ralentie par la friction dans le ou lesdits tubes centraux, ce qui y diminue la force centrifuge et génère un reflux central (41) quasiment non rotatif le long de l'axe de symétrie cylindrique (1). Suivant un mode préféré de l'invention, le ou lesdits tubes centraux sont courts, de préférence d'une longueur inférieure à leur diamètre, et se terminent par un conduit à symétrie cylindrique (21), qui permet d'évacuer tangentiellement les fluides sortant centralement, afin de diminuer le ralentissement de la vitesse de rotation desdits fluides sortant centralement. Ledit conduit à symétrie cylindrique (21) est délimité d'un côté par la surface évasée (20) de l'extrémité dudit tube central et de l'autre côté par un disque latéral (22) dont la partie centrale a une surface bombée (26) qui dévie progressivement les fluides, dits fluides déviés (28) sortant centralement, sans entraver leur rotation autour de l'axe de symétrie (1). Le profil de ladite surface bombée (26) dudit disque latéral (22) a de préférence la forme d'un cône de profil courbe dont la pointe (40) peut être arrondie et dont la base est de préférence évasée.

Suivant un mode particulier de l'invention, ladite pointe (40) de la surface bombée (26) dudit disque latéral (22) pénètre dans ledit tube central, jusqu'à l'entrée et de préférence au-delà de l'entrée dudit tube central, afin de repousser ledit reflux central (41) dans la chambre cyclonique.

Ledit conduit à symétrie cylindrique (21) est fermé par une paroi circulaire (23), de préférence étroite, dont la largeur intérieure (49) est inférieure au rayon de l'entrée dudit tube central et de préférence inférieure à la moitié dudit rayon. Ladite paroi circulaire (23) est pourvue d'au moins 2 ouvertures de sortie tangentielle (24) permettant d'évacuer tangentiellement, dans le sens de rotation des fluides, lesdits fluides déviés (28) tournant autour de l'axe de symétrie cylindrique (1) dans ledit conduit à symétrie cylindrique (21).

Un nombre élevé d'ouvertures de sortie tangentielles (24) permet de réduire la turbulence périodique indésirable dans ledit tube central, transmise à l'intérieur de la chambre cyclonique. Il est donc souhaitable d'en avoir au moins 4 et de préférence au moins 6. La somme des sections desdites ouvertures de sorties tangentielles (24) est plus petite et de préférence au moins deux fois plus petite que la section de l'entrée dudit tube central, et de préférence suffisamment petite pour que la vitesse moyenne d'évacuation des fluides au travers desdites ouvertures de sorties tangentielles (24) soit plus grande que la vitesse moyenne totale des fluides sortant centralement (6) à l'entrée dudit tube central, lorsque le dispositif est en fonctionnement. De cette manière, la vitesse de rotation autour de l'axe de symétrie (1) des fluides sortants centralement n'est pas ralentie par ledit tube central.

La grande énergie cinétique des fluides évacués peut être récupérée partiellement, par exemple s'ils alimentent directement une turbine. Suivant un mode préféré de ce mode particulier de l'invention, lesdites ouvertures de sorties tangentielles (24) sont prolongées par des conduits évasés (42) dont la section de sortie est au moins deux fois et de préférence au moins trois fois plus grande que la section desdites ouvertures de sorties tangentielles (24), pour ralentir les fluides évacués, afin de récupérer une partie de leur énergie cinétique. Lesdits conduits évasés (42) peuvent être reliés à un ou plusieurs collecteurs (89).

Suivant un autre mode particulier de l'invention, le dispositif d'alimentation de fluides secondaires comprend deux chambres contiguës d'alimentation centrale, (15.1) et (14), permettant d'injecter des fluides secondaires différents, l'un gazeux et l'autre liquide, afin de pulvériser dans le dit anneau rotatif (44.1) de fluides secondaires des gouttelettes du liquide secondaire. Les dites gouttelettes dudit liquide secondaire peuvent être utilisées pour réagir avec lesdits flux de fluides sortants et/ou lesdites particules solides entraînées par lesdits flux de fluides sortants.

Suivant un autre mode particulier de l'invention, ledit tube central (7) d'évacuation des fluides contient un ou plusieurs tubes concentriques (80) qui pénètrent plus ou moins loin à l'intérieur de la chambre cyclonique. Suivant ce mode particulier de l'invention, ce ou ces dits tubes concentriques peuvent servir à évacuer ou à alimenter des fluides centralement ou servir à pulvériser un liquide (86).

Suivant un mode particulier de l'invention, le dispositif d'évacuation des particules solides comprend au moins une jambe de décantation (91) traversant ladite paroi périphérique et permettant l'accumulation des particules solides (63) sous l'effet de leur inertie et de la force de gravitation lorsque le dispositif est en fonctionnement. Ladite jambe de décantation peut être munie d'une vanne d'entrée et de sortie s'ouvrant alternativement ou d'une vanne rotative permettant l'évacuation des particules solides accumulées, au rythme souhaité et sans entraîner les fluides tournant dans ladite chambre cyclonique.

La présente invention se rapporte également à l'utilisation du dispositif selon l'invention par des procédés de transformation de particules solides réagissant au contact des fluides traversant la chambre cyclonique et par des procédés de transformation de fluides traversant la chambre cyclonique au contact des particules solides, caractérisés en ce qu'un fluide secondaire injecté par un dispositif selon l'invention est utilisé pour refouler les particules solides entraînées par lesdits flux de fluides sortant le long desdites parois latérale de la chambre cyclonique.

Les procédés de transformation de particules solides réagissant au contact des fluides traversant la chambre cyclonique comprennent, à titre indicatif de manière non limitative, la combustion, la gazéification, la pyrolyse, la torréfaction, le séchage, l'imprégnation, l'enduisage, l'enrobage, la polymérisation, l'oxydation et la réduction desdites particules solides. Les procédés de transformation de fluides traversant la chambre cyclonique au contact des particules solides comprennent, à titre indicatif de manière non limitative, le craquage, la déshydrogénation, la transformation d'alcool en oléfines, la disproportionation, l'oxydation et la réduction desdits fluides. Les particules solides peuvent être ou contenir des catalyseurs.

Le dispositif d'injection de fluides secondaires et les modes préférés d'évacuation centrale des fluides d'une chambre cyclonique, selon l'invention, permet d'augmenter la vitesse de rotation des fluides dans la zone libre du lit fluidifié rotatif, sans augmenter significativement la vitesse de rotation dudit lit fluidifié rotatif. Il est donc particulièrement indiqué pour les procédés où la vitesse de rotation des particules solides le long de la paroi circulaire est limitée par des contraintes mécaniques ou physiques, comme par exemple l'abrasion, la trop grande masse du lit fluidifié rotatif, la fragilité des particules solides ou d'autres effets indésirables.

L'amélioration du refoulement des particules solides entraînées par les flux de fluides sortant le long des parois latérales permet d'utiliser des particules solides plus petites et donc plus réactives. La plus grande réactivité des particules solides permet d'augmenter le débit et donc de diminuer les temps de séjour des fluides tournant dans la chambre cyclonique. La diminution des temps de séjour des fluides permet d'augmenter la température de réaction et/ou de diminuer les réactions secondaires indésirables, grâce au refroidissement rapide desdits flux de fluides sortants par lesdits fluides secondaires. Par exemple le craquage catalytique d'hydrocarbures à hautes températures pour obtenir des oléfines génère de nombreuses réactions secondaires indésirables. Les temps de séjour desdits hydrocarbures dans une chambre cyclonique peuvent être inférieurs à 0,1 seconde à comparer à généralement plus d'une seconde dans les craqueurs catalytiques fluidifiés existants.

La présente invention se rapporte aussi aux procédés de transformation de particules solides et de fluides tournant dans une chambre cyclonique comprenant un dispositif d'injection de fluide secondaire selon l'invention, caractérisés en ce qu'au moins un dit fluide secondaire réagit avec au moins un des flux de fluides sortant ou avec les particules solides entraînées par ledit flux de fluide sortant le long d'une dite paroi latérale ou de séparation.

Le terme "réagir" comprend, à titre indicatif et de manière non limitative, les réactions chimiques, comme par exemple des réactions d'oxydation et de réduction, et les réactions physiques, comme par exemple le refroidissement, l'imprégnation, l'enduisage et l'enrobage.

Par exemple, la combustion de matières carbonées dans un lit fluidifié rotatif doit parfois être incomplète en raison de la limitation de la température de combustion pour éviter ou limiter la formation de scories. L'oxygène contenu dans un fluide secondaire peut achever la combustion qui génère des températures très élevées sans augmenter significativement la température des particules solides tournant le long de la paroi périphérique de la chambre cyclonique.

Un mode particulier de l'invention est un procédé de combustion de matières carbonées, fluides ou solides, tournant dans une chambre cyclonique, caractérisé en ce que les fluides injectés tangentiellement (73) au travers de la paroi circulaire (4) ne contiennent pas suffisamment d'oxygène pour obtenir une combustion complète desdites matières carbonées et en ce qu'au moins un dit fluide secondaire alimenté par un dispositif selon l'invention contient l'oxygène nécessaire pour achever l'oxydation desdits flux de fluides sortant le long d'une paroi latérale.

La présente invention se rapporte également aux procédés de transformation de particules solides et de fluides tournant dans une chambre cyclonique, caractérisée en ce qu'il comprend au moins deux dites chambres contiguës d'alimentation centrale, l'une injectant un liquide secondaire et l'autre un gaz secondaire, par un des dispositifs selon l'invention, ledit liquide secondaire réagissant avec au moins un des flux de fluides ou avec les particules solides entraînées par ledit flux de fluides sortant le long d'une paroi latérale ou de séparation.

Par exemple, pour obtenir un refroidissement très rapide, ledit liquide secondaires peut être un liquide de refroidissement, entraîné et dispersé par ledit gaz secondaire dans ledit tourbillon toroïdal secondaire. Les gouttelettes de liquide peuvent se mélanger au flux de gaz chauds, longeant les parois latérales vers le tube central et refroidir le gaz chaud en s'évaporant avant même que lesdits gaz chauds aient quitté la chambre cyclonique.

Ledit liquide secondaire peut aussi être utilisé pour réagir avec les particules solides entraînées par lesdits flux de fluides sortant (30) le long de la paroi latérale, comme par exemple les imprégner ou les enduire ce qui les alourdit et améliore le refoulement desdites particules solides vers ladite paroi périphérique.

La présente invention se rapporte également aux procédés de transformation de fluides et de particules solides comprenant la transformation alternée des particules solides tournant dans une chambre cyclonique divisée en 2 sections transversales, A et B, séparées par une paroi de séparation (53), avec différentes réactions dans chaque section, par exemple, une réaction endothermique dans une section et exothermique dans l'autre section, caractérisé en ce qu'au moins un dit fluide secondaire injecté par un dispositif selon l'invention est utilisé pour réagir avec au moins un des flux de fluides sortant ou avec les particules solides entraînées par ledit flux de fluide sortant le long d'une dite paroi latérale ou de séparation.

Par exemple, le carbone qui se dépose sur les catalyseurs lors du craquage catalytique d'hydrocarbures dans une section peut être oxydé dans l'autre section ou par exemple les catalyseurs contenant des oxydes métalliques pour la déshydrogénation par oxydoréduction de l'éthylbenzène en styrène ou pour l'oxydation de matières carbonées peuvent être réduits dans une section et ré oxydés dans l'autre section. Les fluides secondaires peuvent, par exemple, oxyder les fluides sortants ou les refroidir rapidement pour éviter les réactions secondaires.

### Brève description des dessins

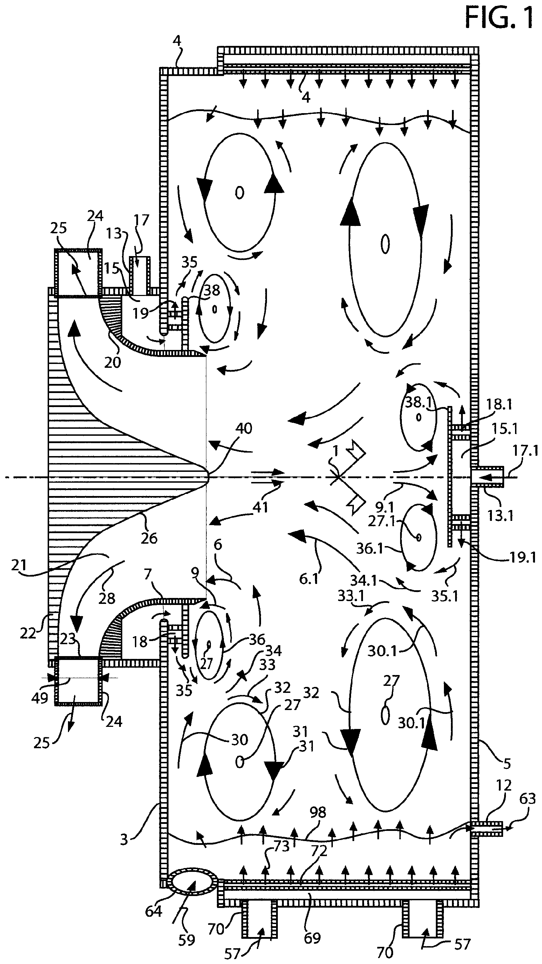

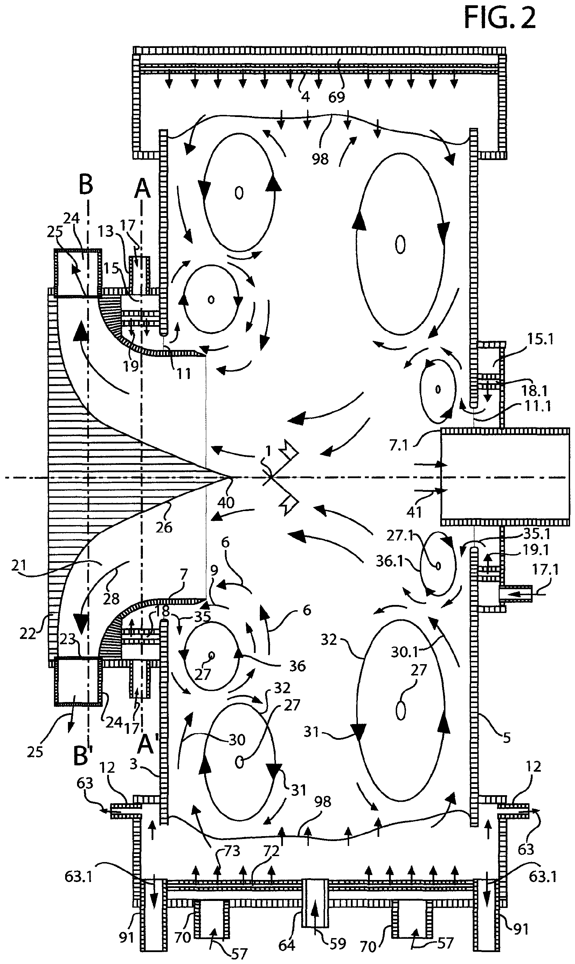

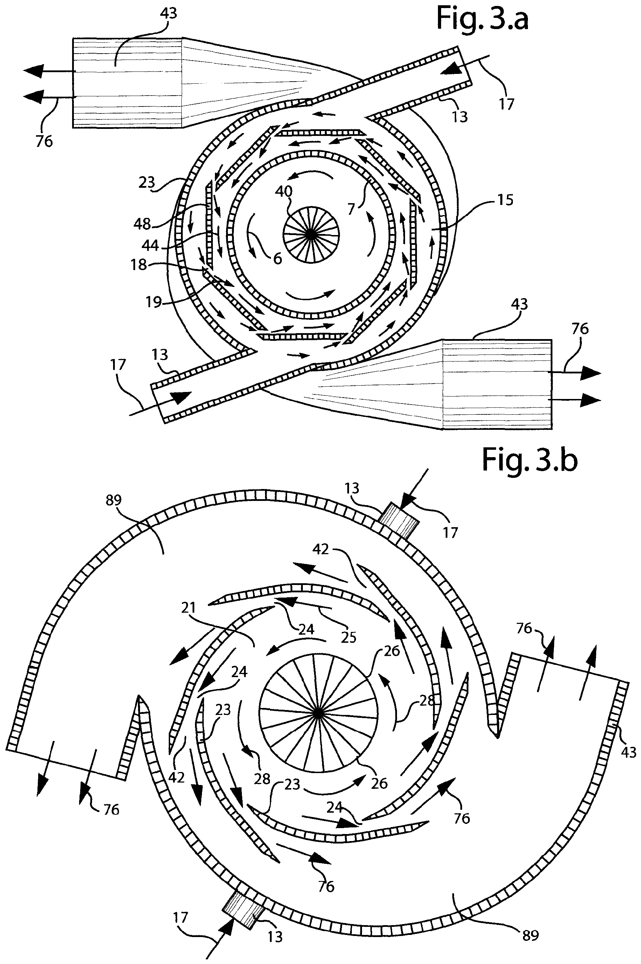

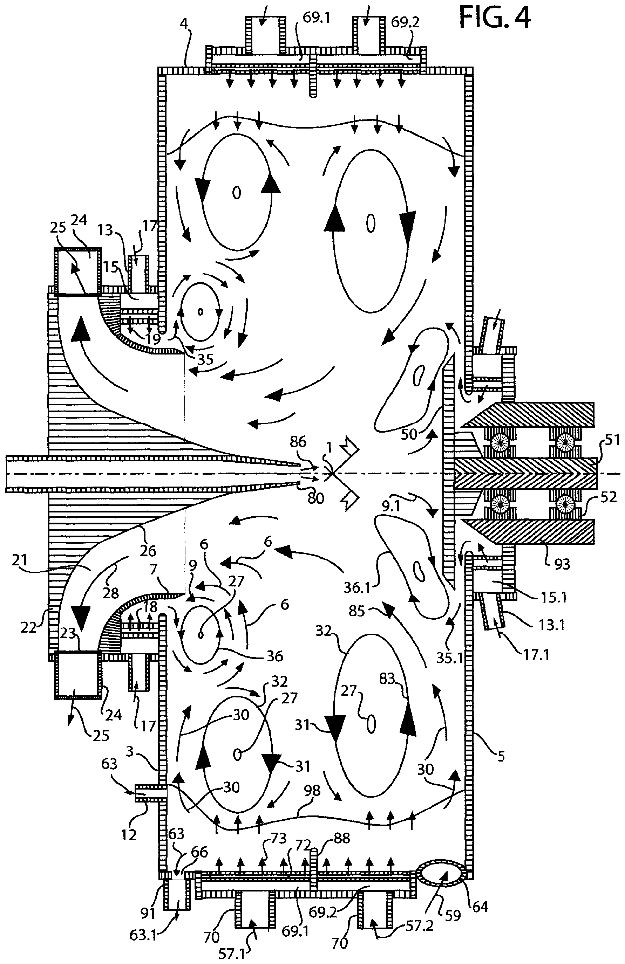

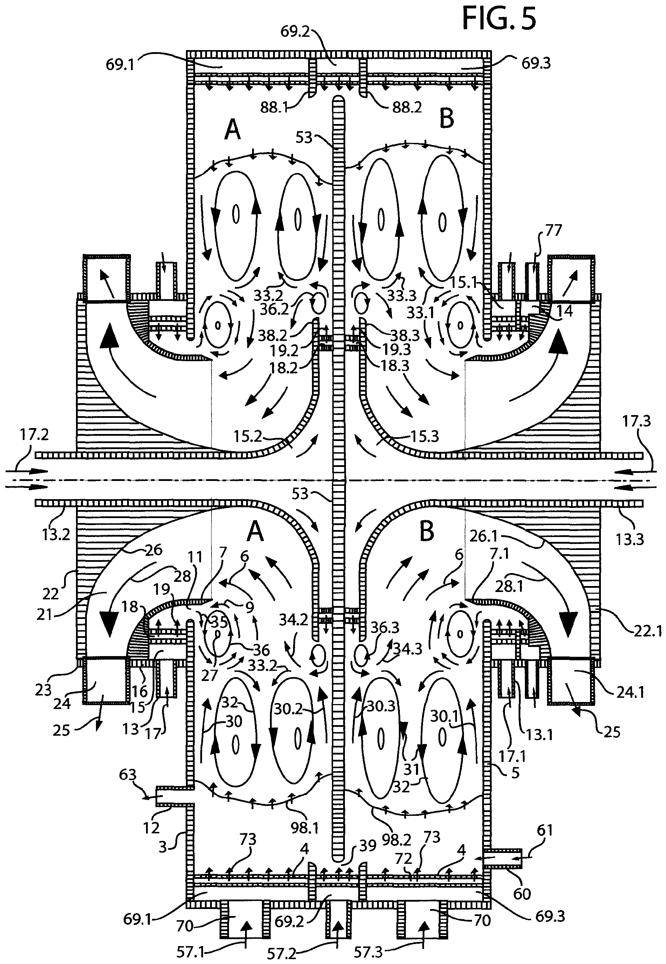

**La** **figure 1** montre la section axiale d'un exemple de dispositif d'injection de fluides secondaires le long des parois latérales dans la zone libre d'un lit fluidifié rotatif dans une chambre cyclonique et un mode préféré d'évacuation des fluides tournant dans ladite chambre cyclonique.
**La** **figure 2** montre la section axiale d'un autre exemple de dispositif d'injection de fluides secondaires dont lesdites ouvertures d'injection (18) et (18.1) sont situées à l'extérieur de la chambre cyclonique, le long des parois latérales (3) et (5) et avec un tube central d'évacuation des fluides de chaque côté latéral de ladite chambre cyclonique.
**La** **figure 3****.a** montre la section transversale du dispositif d'injection de fluides secondaires, suivant la coupe AA' de la figure 2.
**La** **figure 3****.b** montre la section transversale du dispositif d'évacuation centrale des fluides tournant dans la chambre cyclonique, suivant la coupe BB' de la figure 2.
**La** **figure 4** montre la section axiale d'un autre exemple des dispositifs d'injection de fluides secondaires avec un disque rotatif latéral (50) à proximité dudit côté latéral (5) d'une chambre cyclonique.
**La** **figure 5** montre la section axiale d'une chambre cyclonique divisée en deux sections transversales A et B par une paroi de séparation (53) ayant des dispositifs d'injections de fluides secondaires le long de ses deux côtés et d'un dispositif d'injection avec 2 chambres contiguës (15.1) et (14) d'alimentation centrale pour l'injection de fluides secondaires différents.

### Définitions

**Chambre cyclonique ou tourbillonnaire,** également appelées **chambre "vortex",** représente une chambre de forme circulaire, généralement cylindrique, dans laquelle un ou plusieurs fluides sont alimentés tangentiellement afin de faire tourner rapidement à l'intérieur de ladite chambre des particules solides alimentées avec lesdits fluides ou séparément. Lesdits fluides sont évacués centralement après avoir été séparés par la force centrifuge desdites particules solides, qui sont évacuées séparément. Un exemple particulier de chambre cyclonique est un simple **séparateur cyclonique** également appelé **cyclone.** Les séparateurs cycloniques sont généralement très allongés, avec une longueur de préférence au moins trois fois leur diamètre ; leur axe de symétrie cylindrique est généralement vertical et leur partie inférieure a généralement la forme d'un cône très allongé au bas duquel les particules solides sont collectées par gravitation.

**Chambre cyclonique avec lit fluidifié rotatif** est une chambre cyclonique dont la paroi périphérique est pourvue d'ouvertures d'alimentation de fluides permettant d'injecter tangentiellement un fluide qui traverse un lit fluidifié de particules solides en suspension dans ledit fluide et tournant le long de ladite paroi périphérique. La distance entre les deux parois latérales est généralement petite, de préférence inférieure au diamètre et même au rayon de la paroi périphérique. La distance entre lesdites ouvertures d'alimentation de fluides est généralement inférieure au rayon et de préférence à la motié du rayon de la paroi périphérique. La récupération des particules solides se fait de préférence le long ou à proximité de la paroi périphérique.

Fluide: représente un liquide ou un gaz ou un mélange des deux. Lorsque la chambre cyclonique est équipée de dispositif de pulvérisation de gouttelettes d'un liquide, les autres fluides sont généralement des gaz.

**Flux de fluides** : est l'écoulement turbulent de fluides, pouvant entraîner des particules solides, dans des directions moyennes approximatives, représenté, à titre purement indicatif, par des lignes fléchées.

**Tourbillon toroïdal** ou **tourbillon de type toroïdal** se rapporte à des tourbillons complexes qui tournent à la fois autour de l'axe de symétrie cylindrique (1) et sur eux-mêmes, c'est-à-dire autour d'une zone circulaire centrale (27), de forme approximativement toroïdale. Les sections de ces tourbillons complexes sont schématisées par des courbes fermées, qui sont une représentation très simplifiée et à titre purement indicatif de la circulation turbulente complexe des fluides tournant à l'intérieure des chambres cycloniques.

**Zone libre:** lorsqu'un lit fluidifié rotatif est formé dans une chambre cyclonique, les particules solides en suspension dans le fluide, qui traverse ledit lit fluidifié rotatif, sont concentrées par la force centrifuge dans une zone relativement mince le long de la paroi périphérique, tandis que la zone centrale, appelée zone libre, ne contient que peu de particules solides, car la force de Coriolis y augmente la force centrifuge et refoule les particules solides vers la paroi périphérique.

**Surface libre du lit fluidifié rotatif** aussi appelée surface de séparation est la surface séparant le lit fluidifié rotatif de la zone libre. En réalité cette surface est floue et turbulente. Sa section est ci-après représentée par une ligne ondulée (98).

**Direction principalement tangentielle** en un point quelconque est une direction dont la composante tangentielle à la circonférence centrée sur l'axe de symétrie cylindrique (1) et passant par ce point est plus grande que l'ensemble des autres composantes.

### Description détaillée

La figure 1 montre la section axiale d'une chambre cyclonique entourée d'une paroi périphérique (4) et de 2 parois latérales (3) et (5), comprenant
- un dispositif d'alimentation de particules solide (59), pouvant être entraînées par un fluide, par un tube (64) traversant ladite paroi périphérique et un dispositif d'évacuation des particules solides (63) par un tube (12) traversant la paroi latérale (5) à une certaine distance de ladite paroi périphérique;
- un dispositif d'alimentation de fluide (57) dans une chambre d'alimentation (69) entourant ladite paroi périphérique, par des tubes (70), permettant d'injecter lesdits fluides au travers d'un grand nombre d'ouvertures (72), représentées sous la forme de fentes longitudinales, dans une direction (73) principalement tangentielle, c'est-à-dire principalement perpendiculaire au plan de la figure, de ladite paroi périphérique, permettant la formation d'un lit fluidifié rotatif;
- un dispositif d'évacuation des fluides (25) par un tube central (7) traversant la paroi latérale (3) ;
caractérisé en ce qu'il comprend:
- un dispositifs d'alimentation de fluides secondaires (17) et (17.1) par les tubes (13) et (13.1) au travers de chambres d'alimentation centrale (15) et (15.1), disposées le long de chaque paroi latéral (3) et (5), l'une autour dudit tube central (7) et l'autre autour de l'axe de symétrie cylindrique (1) et pourvues d'ouvertures, représentées par les fentes (18) et (18.1), réparties autour dudit axe de symétrie cylindrique, disposées à l'intérieur de la chambre cyclonique dans cet exemple, permettant d'injecter lesdits fluides secondaires (17) et (17.1) dans des directions principalement tangentielles (19) et (19.1) le long desdites paros latérales (3) et (5), à l'intérieur de ladite zone libre, afin de former des anneaux rotatifs de fluide, derrière des parois de guidage (38) et (38.1).

Lorsque la chambre cyclonique est en fonctionnement sans le dispositif d'injection de fluides secondaires selon l'invention, la force centrifuge des fluides tournant autour de l'axe de symétrie cylindrique (1) concentre la plus grande partie des particules solides (63) dans le lit fluidifié rotatif dont la surface de séparation avec ladite zone libre est représentée par la ligne ondulée (98) le long de ladite paroi périphérique. Lesdites particules solides (63) sont évacuées par le tube (12), dont la distance à ladite paroi périphérique détermine la distance moyenne de ladite surface libre et de ladite paroi périphérique et donc l'épaisseur moyenne du lit fluidifié rotatif.

La friction le long desdites parois latérales diminue la vitesse de rotation des fluides autour de l'axe de symétrie cylindrique (1), ce qui diminue la force centrifuge et favorise l'entraînement de particules solides par les flux de fluides sortant (30) et (30.1) le long desdites parois latérales. La force centrifuge générée par l'injection tangentielle (73) des fluides est plus élevée à une certaine distance des parois latérales et peut générer un reflux de fluides (31) vers ladite paroi périphérique, formant ainsi des tourbillons de type toroïdal (32) et (32.1) de fluides tournant à la fois autour de l'axe de symétrie cylindrique (1) et autour d'une zone circulaire (27) au centre des sections axiales desdits tourbillons de type toroïdal. Ce reflux de fluide ralentit la vitesse de rotation des fluides et augmente l'entraînement des particules solides vers la sortie centrale par les flux de fluides sortant (30) et (30.1) le long des parois latérales.

Lorsque le dispositif selon l'invention alimente lesdits fluides secondaires à une pression suffisante, la force centrifuge générée par la grande vitesse de rotation desdits anneaux rotatifs de fluides secondaires, génère un reflux de fluides secondaires (35) et (35.1) le long desdites parois latérales, éloignant desdites parois latérales et accélérant la vitesse de rotation desdits flux de fluides sortants (30) et (30.1), dont une partie, dite partie refoulée (33) et (33.1), contenant la plupart des particules solides entraînées par les dits flux de fluides sortants, est refoulée vers le milieu de ladite paroi périphérique. L'autre partie, dite partie évacuée (34) et (34.1), ne contenant pratiquement plus de particules solides, est évacuée vers le tube central (7) en se mélangeant au fluide secondaire. Une petite partie, dite partie recyclée (9) et (9.1), attirée par la dépression générée par la force centrifuge desdits anneaux rotatifs de fluides secondaires, est recyclée en se mélangeant au reflux de fluides secondaires (35) et (35.1), formant ainsi un tourbillon, dit tourbillon toroïdal secondaire (36) et (36.1) tournant à la fois autour de l'axe de symétrie cylindrique (1) et autour d'une zone circulaire (27) à l'intérieur des sections axiales dudit tourbillon toroïdal secondaire. Ces sections et ces tourbillons sont de formes variables. Ils sont schématisés à titre purement indicatif.

La vitesse de rotation desdites parties refoulées desdits flux de fluides sortant le long des parois latérales, chargées de particules solides, est augmentée par lesdits tourbillons toroïdaux secondaires, ce qui améliore la séparation des particules solides des fluides traversant le lit fluidifié rotatif.

La pression d'injection des dispositifs d'alimentation de fluides secondaires doit pouvoir être suffisamment élevée pour que la vitesse d'injection des fluides secondaires puisse être plus grande et de préférence au moins une fois et demi plus grande que la plus grande vitesse de rotation des fluides sortant centralement (6) de ladite chambre cyclonique, lorsqu'elle est en fonctionnement sans le dispositif selon l'invention.

Le moment cinétique de rotation transféré par lesdits tourbillons toroïdaux secondaires aux flux de fluides sortants dépend de la vitesse d'injection, et donc de la pression d'injection, ainsi que du débit desdits fluides secondaires, et donc de la section totale des ouvertures d'injection (18). Ces grandeurs peuvent être choisies en fonction des besoins et des contraintes du procédé utilisant ce dispositif. Si le rôle des fluides secondaires est uniquement l'amélioration de la qualité de la séparation des fluides et des particules solides fines, un petit débit, avec de grandes vitesses d'injection et donc une pression d'injection élevée au travers d'ouvertures d'injection de petites sections est préférable. Toutefois le transfert de moment cinétique de rotation devient inefficient lorsque les différences de vitesses sont très grandes. Il est donc souhaitable d'avoir un débit desdits fluides secondaires suffisamment élevé, de préférence dans chacune desdites chambres d'alimentation centrale, d'au moins 5% et de préférence au moins 8% du débit des fluides traversant la chambre cyclonique.

Lorsque les fluides sont évacués par un tube central (7) ordinaire, la friction le long et à l'extrémité du tube freine la vitesse de rotation des fluides et donc la force centrifuge à l'intérieur dudit tube central, ce qui diminue la dépression centrifuge le long de l'axe de symétrie et génère un gradient de pression vers ladite chambre cyclonique et donc un reflux central (41) quasiment non rotatif, qui ralentit la vitesse de rotation des fluides dans ladite chambre cyclonique.

Les tourbillons toroïdaux secondaires (36) et (36.1) augmentent la vitesse de rotation autour de l'axe de symétrie (1) des fluides sortants centralement (6) dans le tube central (7), et donc aussi les pertes dues à la friction à l'intérieur du tube central (7) et le reflux central (41) indésirable, vers ladite chambre cyclonique. Afin de diminuer ces pertes de vitesse de rotation, le tube central (7) d'évacuation des fluides sortants centralement (6) est court, de préférence plus petit que le diamètre de son entrée. Son extrémité extérieure est évasée et se termine par un conduit à symétrie cylindrique (21), délimité d'un côté par la surface évasée (20) dudit tube central et de l'autre côté par la surface centrale bombée (26) d'un disque latéral (22) fermant la sortie axiale dudit tube central.

Ledit conduit à symétrie cylindrique (21), qui permet de dévier les fluides sortant centralement (6), dits fluides déviés (28), sans empêcher leur rotation autour de l'axe de symétrie (1), en transformant leur vitesse axiale (29) en vitesse radiale, se termine par une paroi circulaire (23), comprenant au moins 2 et de préférence au moins 6 ouvertures de sorties tangentielles (24) permettant d'évacuer les fluides (25) dans une direction principalement tangentielle au travers de ladite paroi circulaire (23). Ladite surface bombée (26) a un profil de type conique, avec une pointe (40) arrondie et un évasement à la base, choisi pour que les sections dudit conduit à symétrie cylindrique (21) soient inférieures à la section d'entrée dudit tube central (7), afin de dévier progressivement les fluides sortants, sans les ralentir.

La largeur intérieure (49) de ladite paroi circulaire (23) est inférieure au rayon et de préférence à la moitié du rayon de l'entrée dudit tube central (7) pour que la vitesse radiale de sortie des fluides évacués (25) au travers de ladite paroi circulaire (23) soit relativement grande. La somme des sections desdites ouvertures de sorties tangentielles (24) est inférieure à la moitié de la section d'entrée du tube central (7) et de préférence suffisamment petite pour que la vitesse de sortie desdits fluides évacués (25) soit supérieure à la vitesse moyenne totale des fluides sortants centralement (6) à l'entrée du tube central (7).

Ladite pointe (40) de ladite surface bombée pénètre dans ledit tube central (7), de préférence jusqu'au-delà de l'entrée dudit tube central (7), afin de repousser l'initiation du reflux central (41) dans la chambre cyclonique. Ledit reflux central (41), quasiment non rotatif, est aspiré et recyclé (9.1) par la dépression générée par ledit tourbillon toroïdal secondaire (36.1) qui lui transfère une partie de son moment cinétique de rotation avant d'être évacué par le tube central (7). Ce transfère de moment cinétique de rotation réduit l'influence négative dudit reflux central sur la vitesse de rotation des fluides à l'intérieur de la chambre cyclonique.

L'évacuation centrale des fluides selon le mode préféré de l'invention décrit dans cet exemple, permet ainsi de réduire et même d'inverser l'influence négative du tube central d'évacuation des fluides sur la vitesse de rotation des fluides à l'intérieur dudit cyclone.

La chute de pression statique des fluides évacués (25) à grande vitesse peut être partiellement récupérée en aval des ouvertures de sorties tangentielles (24), à l'aide de conduits de sorties évasés (42), qui permettent de récupérer une partie de leur pression dynamique, comme le montre la figure 3.b.

La figure 2 montre la section axiale d'un autre exemple des dispositifs d'injection de fluides secondaires dont lesdites ouvertures d'injection (18) et (18.1) sont situées à l'extérieur de la chambre cyclonique, le long des parois latérales (3) et (5) et avec un tube central (7) et (7.1) d'évacuation de fluides de chaque côté latéral de ladite chambre cyclonique. Dans cet exemple, l'alimentation des particules solides (59) se fait au travers du tube (64) traversant le milieu de la paroi périphérique (4). Elles sont entraînées dans un mouvement de rotation par le fluide (57) injecté dans une direction principalement tangentielle (73) dans la chambre cyclonique. Elles s'ajoutent aux particules solides refoulées au milieu de la chambre cyclonique et elles sont poussées axialement par la force centrifuge vers les parois latérales (3) et (5).

L'évacuation des particules solides lourdes se fait par 2 jambes de décantation (91) situées à proximité des parois latérales (3) et (5). Des vannes rotatives ou des paires de vannes s'ouvrant alternativement, non représentés sur la figure, permettent d'absorber les différences de pression sans entraîner les fluides tournant dans la chambre cyclonique et de contrôler le débit des particules solides lourdes.

Les autres particules solides, principalement les plus fines ou les plus légères, sont évacuées par les tubes de sortie (12) qui déterminent l'épaisseur du lit fluidifié rotatif dont la surface libre est représentée par la ligne ondulée (98). La séparation desdites autres particules solides et du flux de fluide qui les entraîne dans les tubes (12) peut se faire par un dispositif adéquat, comme un cyclone ou un filtre.

Les ouvertures d'injection (18) et (18.1) des dispositifs d'alimentation des fluides secondaires (17) et (17.1) sont situées, dans cet exemple, à l'extérieur de la chambre cyclonique. Lesdits anneaux rotatifs de fluides secondaires, formés derrière lesdites parois latérales, pénètrent dans la chambre cyclonique au travers des ouvertures annulaires (11) et (11.1), de préférence étroites, entre lesdits tubes centraux (7) et (7.1) et lesdites parois latérales (3) et (5), qui remplissent la fonction des parois de guidage (38) et (38.1) de la figure 1. Cette séparation permet d'homogénéiser lesdits anneaux rotatifs de fluides secondaires avant d'entrer en contact avec les fluides tournant dans la chambre cyclonique, afin d'éviter de générer une turbulence indésirable.

Le deuxième tube central (7.1) d'évacuation des fluides est relativement petit dans cet exemple afin d'évacuer principalement le reflux central (41), provenant de la zone quasiment non rotative longeant l'axe de symétrie cylindrique (1). Ce tube central (7.1) peut être semblable au tube central (7), comme dans la figure 5.

**La** **figure 3****.a** montre la section transversale, suivant la coupe AA' de la figure 2, du dispositif d'injection de fluides secondaires, (17), alimentés sous pression par les tubes d'alimentation (13) dans la chambre d'alimentation centrale (15) et injectés au travers de 8 ouvertures d'injection (18), délimitées par 8 plaquettes successives (48). Ces fluides secondaires injectés dans une direction principalement tangentielle (19) et à grande vitesse autour du tube central (7) forment un anneau rotatif (44) de fluide secondaire entre lesdites plaquettes successives (48) et ledit tube central (7) à l'extérieur de la paroi latérale (3), avant de pénétrer dans ladite chambre cyclonique. Ledit anneau rotatif est représenté respectivement par les nombres (44.1), (44.2) et (44.3) lorsqu'il se rapporte aux fluides secondaires (17.1), (17.2) et (17.3).

Les flux de fluides sortant centralement (6) tournent à l'intérieur du tube central (7) autour de la section de la pointe (40) de ladite surface bombée dudit disque latéral. Ils sont évacués par deux tubes de sortie (43), montrés en arrière plan à titre indicatif.

**La** **figure 3****.b** montre la section transversale, suivant la coupe BB' de la figure 2, du dispositif d'évacuation centrale des fluides, suivant un mode préféré de l'invention. Les fluides déviés (28) tournent autour de la surface bombée (26), à l'intérieur du conduit à symétrie cylindrique (21), et sont évacués (25) à grande vitesse, au travers de 6 ouvertures de sorties tangentielles (24), de petites sections, reliées à 2 collecteurs (89) par des conduits évasés (42), dont les sections de sortie sont de préférence au moins trois fois plus grandes que les sections des sorties tangentielles (24), afin de récupérer une partie de la pression dynamique des fluides évacués (25). Les fluides ralentis (76) sont évacués par les deux tubes (43).

Si les sections des ouvertures des sorties tangentielles (24) sont assez petites, ce dispositif permet de maintenir et même d'augmenter la vitesse de rotation des fluides déviés (28) et donc des fluides sortant centralement (6) dans le tube central (7), afin d'avoir une influence positive sur la vitesse de rotation des fluides dans ladite chambre cyclonique.

Les tubes d'alimentation (13) de fluides secondaires (17) sont montrés en arrière plan à titre indicatif.

**La** **figure 4** montre un autre exemple d'une chambre cyclonique équipée des dispositifs d'injection de fluides secondaires, selon l'invention, avec un disque rotatif latéral (50), à proximité de la paroi latérale (5), guidant le reflux de fluide secondaire (35.1) et supporté par un dispositif symbolisé par un arbre rotatif (51) et des roulements à billes (52) tenus par un support fixe (93), permettant de faire tourner ledit disque rotatif à une vitesse suffisante pour accélérer la vitesse de rotation des fluides tournant dans la chambre cyclonique à proximité du disque rotatif. La plus grande force centrifuge générée par le disque rotatif augmente l'intensité du tourbillon toroïdal (36.1) qui refoule les particules solides entraînées par le flux de fluides sortants (30.1) le long de la paroi latérale (5). Le diamètre et la vitesse de rotation dudit disque rotatif, sont choisis en fonction des besoins du procédé utilisant ce dispositif.

Dans cet exemple, un tube (64) alimente des particules solides (59) au travers de ladite paroi périphérique, de préférence entraînées tangentiellement par un fluide, à proximité de la paroi latérale (5). Les fluides (57.1) et (57.2), alimentés par les tubes (70) dans les chambres d'alimentation (69.1) et (69.2) entourant la chambre cyclonique, sont injectés tangentiellement par des ouvertures d'injection, comme, par exemple, des fentes longitudinales d'injection (72), afin de faire tourner lesdits fluides et lesdites particules solides (63) dans la chambre cyclonique.

Les plus grosses particules solides (63.1) sont évacuées au travers d'une ouverture de sortie (66) le long de la paroi circulaire (4) par une jambe de décantation (91), située de préférence à proximité de l'autre paroi latérale (3) et les particules (63) plus légères sont évacuées de préférence par un tube (12) au travers de la paroi latérale (3).

Dans cet exemple particulier, le tube central (7), traversant la paroi latérale (3) opposée audit disque rotatif latéral (50), entoure un tube concentrique (80), qui pénètre dans la chambre cyclonique, à proximité du disque rotatif latéral (50). Il permet d'alimenter centralement un fluide (86), qui peut, par exemple, être pulvérisé sous la forme de gouttelettes d'un liquide servant, par exemple, au refroidissement des fluides ou à l'imprégnation des particules solides tournant dans la chambre cyclonique.

Cet exemple comprend également une paroi annulaire de séparation (88) qui permet de diminuer la circulation axiale des particules solides tournant le long de la paroi circulaire (4) entre la dite paroi annulaire de séparation et les parois latérales (3) et (5), en fonction des besoins du procédé utilisant ce dispositif.

La figure 5 montre la section axiale d'un autre exemple d'une chambre cyclonique approximativement symétrique, divisée en deux sections transversales A et B par une paroi de séparation (53), ayant des dispositifs d'injections de fluides secondaires le long des 2 côtés de ladite paroi de séparation.

L'alimentation des fluides de (57.1) à (57.3) se fait au travers de 3 chambres d'alimentation, de (69.1) à (69.3), entourant la chambre cyclonique. L'injection du fluide (57.2) se fait au travers d'ouvertures (72) réparties en face de la périphérie de ladite paroi de séparation (53), située à une petite distance de ladite paroi périphérique, laissant un passage (39), de préférence étroit, permettant le transfert des particules solides d'une dite section transversale à l'autre. Des parois annulaires de séparation (88.1) et (88.2) peuvent guider le fluide (57.2) de part et d'autre de ladite paroi de séparation afin de stripper des fluides qu'elles entraînent, le flux de particules solides, transférées d'une section transversale à l'autre.

Dans cet exemple, le passage (39) couvre l'entièreté de la périphérie de ladite paroi de séparation et les ouvertures (72) d'alimentation du fluide (69.2) sont alignées le long dudit passage (39). La circulation des particules solides entre les deux sections transversales est générée par la turbulence. Cette circulation peut être augmentée à l'aide de passages (39) multiples, disposés alternativement en face de l'amont d'une ouverture (72) d'un côté et de l'aval d'une ouverture (72) de l'autre côté de ladite paroi de séparation.

L'alimentation de particules solides (61) se fait par un tube d'alimentation (60) traversant la paroi latérale (5) et l'évacuation de particules solides (63) par un tube (12) traversant la paroi latérale (3) à une distance de la paroi périphérique (4) qui détermine le niveau moyen de la surface libre (98.1) du lit fluidifié rotatif tournant dans ladite section transversale A. Le niveau moyen de la surface libre (98.2) du lit fluidifié rotatif tournant dans ladite section transversale B est déterminé par la différence de pression entre les zones libres des deux sections transversales. Un dispositif adéquat de contrôle des différences de pressions, non représenté, doit permettre de maintenir la surface libre (98.2) au niveau moyen souhaité.

Les fluides secondaires (17.2) et (17.3) sont alimentés par les tubes (13.2) et (13.3), concentriques aux tubes centraux (7) et (7.1), et injectés par les ouvertures (18.2) et (18.3) dans des directions principalement tangentielles (19.2) et (19.3) derrière les parois de guidage (38.2) et (38.3), afin de former des anneaux rotatifs de fluides secondaires qui accélèrent la vitesse de rotation et repoussent les flux de fluides sortants (30.2) et (30.3) qui entraînent des particules solides le long des surfaces de ladite paroi de séparation (53), afin de refouler vers ladite paroi périphérique lesdites parties refoulées (33.2) et (33.3) qui contiennent la plupart des particules solides entraînées par lesdits flux de fluides sortants (30.2) et (30.3). Lesdites parties évacuées (34.2) et (34.3) ne contiennent pratiquement plus de particules solides.

Dans cet exemple, le dispositif d'alimentation de fluides secondaires autour du tube central (7.1) traversant la paroi latérale (5) comprend une chambres contiguë, (14) à la chambre d'alimentation centrale (15.1) permettant l'alimentation d'un fluide secondaire différent (77) dont l'un peut être gazeux et l'autre liquide, afin de pulvériser ledit liquide à l'intérieur de la chambre cyclonique. Ledit liquide peut réagir, chimiquement et/ou physiquement, avec les fluides sortants (30.1), généralement des gaz, et/ou les particules solides qui sont entraînées par lesdits fluides sortants avant d'être refoulées. Les 3 autres dispositifs d'alimentation des fluides secondaires peuvent aussi disposer d'un tel dispositif de chambre contiguë d'alimentation centrale.

Les différents dispositifs et combinaisons de dispositifs des exemples décrits ci-dessus peuvent être utilisés par des procédés de transformation de particules solides réagissant au contact des fluides tournant dans une chambre cyclonique, comme la combustion, la gazéification, la pyrolyse, la torréfaction, le séchage, l'enrobage, l'enduisage, l'imprégnation, l'extraction de volatiles, l'oxydation, la réduction, etc.... de particules solides, ainsi qu'aux procédés de transformation de fluides tournant dans une chambre cyclonique au contact des particules solides qui peuvent être des catalyseurs et/ou des réactifs comme par exemple le craquage, la disproportionation, la déshydrogénation, l'oxydation, l'oxydoréduction, la polymérisation, etc.... de fluides tournant dans la chambre cyclonique, lesdits procédés de transformation comprenant la séparation desdites particules solides et desdits fluides.

L'utilisation du dispositif d'injection d'air secondaire et d'évacuation centrale des fluides selon l'invention par lesdits procédés de transformation de particules solides et de fluides tournant dans une chambre cyclonique est caractérisée en ce que l'injection de fluides secondaires permet d'améliorer la séparation des particules solides et des fluides sortants, en augmentant la vitesse de rotation des fluides dans la zone libre de la chambre cyclonique, principalement le long des parois latérales et de séparation, sans accélérer significativement la vitesse de rotation dudit lit fluidifié rotatif.

Ce dispositif est donc particulièrement utile pour les procédés où le rapport souhaité entre le débit horaire massique des fluides et la masse du lit fluidifié rotatif est trop petit pour permettre un transfert de moment cinétique de rotation suffisant pour faire tourner le lit fluidifié rotatif à la vitesse nécessaire à l'obtention d'une bonne séparation entre les fluides et les solides ou lorsque les contraintes mécaniques ou physiques, comme par exemple l'abrasion ou la sensibilité des particules solides à la friction, limitent la vitesse de rotation desdites particules solides le long de ladite paroi périphérique, comme, par exemple, la polymérisation de poudres de polyéthylène qui est sensible à la formation de cheveux d'anges.

L'invention se rapporte également aux procédés utilisant les dispositifs d'injection de fluides secondaires selon l'invention, caractérisé en ce qu'au moins un desdits fluides secondaires refroidit ou réagit chimiquement avec au moins un desdits flux de fluides sortants, qui ont réagi avec les particules solides du lit fluidifié tournant dans la chambre cyclonique.

Par exemple les gaz provenant de la gazéification, ou de la pyrolyse ou de la torréfaction ou de l'extraction de volatils des particules solides en suspension dans le lit fluidifié rotatif peuvent être refroidis brutalement par la pulvérisation de liquides secondaires alimentés par des chambres contiguës décrites par la figure 5, afin d'éviter les réactions secondaires indésirables.

La figure 2 permet d'illustrer un exemple de procédé particulier de combustion en deux étapes de matières carbonées, solides ou fluides, comme de la poussière de charbon ou des déchets organiques broyés, avec, par exemple, des gaz enrichis en oxygène, caractérisé en ce qu'une combustion partielle est d'abord réalisée dans le lit fluidifié rotatif de ladite chambre cyclonique et ensuite, l'oxygène dudit fluide secondaire injecté par le dispositif d'injection selon l'invention, termine la combustion des flux de gaz sortants (30) et (30.1).

L'utilisation de gaz enrichi en oxygène permet de réduire le coût de la récupération des produits indésirables comme par exemple le CO2. Cette combustion peut être très rapide, tout en générant des températures très élevées contribuant à la formation de scories. Les chambres cycloniques à lit fluidifié rotatif permettent de contrôler les combustions quasiment explosives grâce au rapport très élevé des débits massiques des gaz divisés par la masse des solides présents dans le lit fluidifié rotatif, pouvant être supérieur à 1000/heure grâce à une force centrifuge de plus d'un ordre de grandeur supérieur à la force de gravité et aussi grâce à la grande surface et à la faible épaisseur du lit fluidifié rotatif.

A titre indicatif, une chambre cyclonique de 1 m³ peut contenir un lit fluidifié rotatif de l'ordre de 100 Kg de poudres et être traversée par un débit massique horaire de gaz sous pression de plus de 200 tonnes par heure, ce qui permet de brûler partiellement de l'ordre de 10 kg par seconde de carbone avec un mélange massique d'environ 2/3 de vapeur et 1/3 d'oxygène pur. Si le lit fluidifié rotatif contient 20% de poussière de charbon, le reste étant des cendres ou des poudres inertes ou oxydantes ou catalytiques, le temps de combustion partielle de la poussière de charbon est de l'ordre de 2 secondes. La combustion des gaz riches en CO peut être achevée au moment de leur sortie de la chambre cyclonique par l'oxygène contenu dans les fluides secondaires, injectés à grande vitesse dans la zone libre de la chambre cyclonique.

Les matières carbonées (59) peuvent, par exemple, être alimentées en étant en suspension ou dissoutes dans un liquide, par exemple de l'eau, à l'aide d'une pompe haute pression. Le mélange peut être préchauffé à une température permettant la vaporisation d'une partie du liquide, afin de générer un mélange de solides, liquide et vapeur, (59), qui peut être injecté tangentiellement, à grande vitesse, dans la chambre cyclonique, par le tube (64). Un gaz contenant une quantité insuffisante d'oxygène pour assurer la combustion complète de tout le carbone contenu dans lesdites matières carbonées, est alimenté par les chambres d'alimentation (69.1) et (69.2) dans la chambre cyclonique préchauffée à une température suffisante pour brûler le carbone en produisant un mélange de CO et de CO2. Les débits peuvent être ajustés afin d'obtenir la température souhaitée, suffisamment élevée pour une combustion rapide et suffisamment basse pour éviter la formation de scories, par exemple de l'ordre de 650°C, dans la partie périphérique de la chambre cyclonique.

Les cendres lourdes (63) peuvent être évacuées au travers de ladite paroi périphérique par les jambes de décantation (91). Les cendres fines ou légères (87) sont évacuées par les sorties latérales (12) ou elles sont entraînées le long des parois latérales (3) et (5), par les flux de fluides sortants (30) et (30.1) et refoulées par les reflux de fluides secondaires (35) et (35.1), formant les tourbillons toroïdaux secondaires (36) et (36.1), vers ladite paroi périphérique.

L'oxygène contenu dans les fluides secondaires (17) et (17.1) peut terminer l'oxydation des gaz partiellement oxydés dans la partie centrale de la chambre cyclonique et dans les tubes centraux, sans que la chaleur dégagée par cette postcombustion, ne surchauffe les cendres formées le long de la paroi circulaire. Les cendres légères qui ont été refoulées par le tourbillon secondaire peuvent être très chaudes et former, dans la partie périphérique de la chambre cyclonique, de petits agglomérats, qui peuvent être évacués par les jambes de décantation (91).

Des parois annulaires de séparation (88), comme illustrées par les figures 4 et 5, peuvent être ajoutées afin de séparer le lit fluidifié rotatif en plusieurs sections, traversées par des fluides de composition et/ou de températures différentes. Par exemple de la vapeur d'eau peut être injectée autour des jambes de décantations afin de stripper les particules lourdes avant de les évacuer.

La petite dimension d'une telle chambre cyclonique permet de travailler à très haute pression et la postcombustion permet d'atteindre les températures nécessaires à la combustion complète des composants indésirables et à la récupération efficiente de l'énergie de la combustion. L'utilisation d'oxygène pur et de matières carbonées en suspension ou dissoute dans de l'eau permet de contrôler la température du lit fluidifié rotatif et de récupérer le CO2 à moindre coût. Enfin la qualité de la séparation des gaz brûlés et des particules solides grâce à l'injection centrale de gaz secondaires très rapides, générant une force centrifuge pouvant être de plusieurs ordres de grandeur supérieure à la pesanteur dans la partie centrale de la chambre cyclonique, peut refouler suffisamment les particules solides fines pour permettre d'alimenter directement une turbine.

Le dispositif décrit par la **figure 4****,** avec ou sans disque rotatif, peut être utilisé, par exemple, pour les procédés d'enrobage, d'enduisage ou d'imprégnation. Le liquide (86) d'enrobage, d'enduisage ou d'imprégnation peut être pulvérisé par le tube (80) sur le disque rotatif (50) tournant à grande vitesse ou à l'aide d'un pulvérisateur, directement dans la zone libre de la chambre cyclonique. L'injection du liquide peut également se faire par des pulvérisateurs traversant une paroi latérale ou la paroi périphérique ou à l'aide d'une chambre d'alimentation centrale contiguë (14), illustrée dans la figure 5.

Le dispositif selon l'invention se rapporte également aux procédés de transformation de particules solides comme l'enduisage, l'enrobage, l'imprégnation, ... caractérisé en ce qu'il comprend au moins une chambre d'alimentation centrale contiguë permettant d'alimenter un liquide secondaire réagissant avec les particules solides entraînées par un dit flux de fluides sortant le long d'une dite paroi latérale ou de séparation.

La force centrifuge pousse les gouttelettes de liquide vers la paroi périphérique où elles peuvent réagir avec les particules solides en suspension dans ledit lit fluidifié rotatif. Les particules solides migrent ensuite vers la paroi latérale (3), de l'autre côté de la chambre cyclonique, le long de la paroi circulaire (4) traversée par les gaz (57.1) et 57.2). La composition et la température de ces gaz sont choisies en fonction des besoins du procédé. Par exemple le gaz (57.2) peut être froid pour retarder le séchage afin d'uniformiser l'enduisage ou être chaud afin d'accélérer le durcissement de l'enduit au fur et à mesure de l'enduisage. Les particules solides sont ensuite évacuées par le tube (12) ou la jambe de décantation (91). Une paroi annulaire (88) peut augmenter le temps de séjour des particules solides dans la section transversale à droite de la chambre cyclonique.

Le dispositif selon l'invention se rapporte également aux procédés de transformation de fluides et de particules solides comprenant la transformation alternée desdites particules solides dans une chambre cyclonique divisée en deux sections transversales suivant un des modes particuliers de l'invention, caractérisé en ce qu'il comprend une réaction endothermique dans une section et une réaction exothermique dans l'autre section et en ce qu'au moins un desdits fluides secondaires réagit avec au moins un desdits flux de fluides sortants.

Le dispositif décrit par la **figure 5** permet d'illustrer les procédés de transformation alternée, par exemple une étape d'oxydation qui régénère les particules solides catalytiques et/ou qui génère la chaleur nécessaire à la deuxième étape, par exemple de craquage catalytique ou de gazéification ou de déshydrogénation qui absorbe de la chaleur. Les particules solides sont alimentées au travers de la paroi latéral (5) par le tube (68) et sont évacuées de l'autre côté latéral par le tube (12). Elles forment un lit fluidifié rotatif traversé d'un côté par le fluide (57.3), par exemple des hydrocarbures, et de l'autre côté par le fluide (57.1), par exemple de l'air. Un fluide de séparation (57.2), par exemple de la vapeur d'eau, peut être utilisé pour stripper les particules solides des fluides qu'elles entraînent avec elles lorsqu'elles passent d'un côté à l'autre.

Lorsque le dispositif est en fonctionnement, les particules solides forment un lit fluidifié rotatif et circulent rapidement d'un côté à l'autre du lit, en transférant leur chaleur et leur moment cinétique de rotation, ce qui contribue à diminuer substantiellement les différences de températures et de vitesses de rotation des deux côtés du lit fluidifié rotatif. Par contre les fluides traversent le lit fluidifié rotatif très rapidement et sont évacués séparément par le dispositif d'évacuation centrale situé de leur côté sans se mélanger, la paroi de séparation assurant une séparation adéquate. Le fluide de séparation (57.2) permet d'améliorer cette séparation en évitant le transfert des autres fluides d'un côté à l'autre. Il peut également être injecté à très grande vitesse afin d'augmenter la vitesse de rotation du lit fluidifié rotatif.

Par exemple, pour un procédé de gazéification, les fluides (57.3) et (57.2) peuvent être de la vapeur d'eau et le fluide (57.1) de l'air. Les particules carbonées sont introduites au travers de la paroi latérale (5) avec d'autres particules solides, comme par exemple de la dolomie, servant à améliorer les transferts de chaleur et pouvant servir aussi de catalyseur ou empêcher la formation de scories.

Le lit fluidifié rotatif est chauffé par la combustion des matières carbonées résiduelles au contact de l'air dans la section A de la chambre cyclonique. Cette chaleur est transférée par le lit fluidifié rotatif dans la section B où les particules carbonées sont introduites, afin d'atteindre la température nécessaire à la gazéification.

Un autre exemple de procédé pouvant utiliser un dispositif semblable à celui décrit par la figure 5 est la déshydrogénation d'hydrocarbures comme l'éthane ou le propane pour obtenir des oléfines ou la déshydrogénation de l'éthylbenzène pour obtenir du styrène par des procédés d'oxydoréduction ou l'oxydation de matières carbonées à l'aide de catalyseurs contenant des oxydes métalliques qui sont réduits dans une section de la chambre cyclonique et ré-oxydés dans l'autre section, qui est traversée par un fluide oxydant. La chaleur produite par l'oxydation dans ladite autre section est transférée par le lit fluidifié rotatif dans la partie où se produit le transfert d'oxygène qui est une réaction endothermique.

## Revendications

1. Dispositif d'injection de fluides secondaires à l'intérieur de la zone libre d'un lit fluidifié rotatif dans une chambre cyclonique fixe comprenant une paroi périphérique (4) entourée de deux parois latérales (3) et (5); un dispositif d'alimentation de fluide (57) au travers d'ouvertures (72), réparties le long de ladite paroi périphérique, dans une direction principalement tangentielle (73); un dispositif d'alimentation et d'évacuation de particules solides; au moins un tube central (7) d'évacuation des fluides tournant dans ladite chambre cyclonique; **caractérisé en ce qu'**il comprend :
- un dispositif d'alimentation de fluides secondaires (17) et (17.1) au travers de chambres d'alimentation centrales (15) et (15.1), situées le long de chacune desdites parois latérales (3) et (5), pourvues d'ouvertures d'injection (18) et (18.1), réparties autour de l'axe de symétrie cylindrique, permettant d'injecter les dits fluides secondaires dans une direction principalement tangentielle (19) et (19.1), afin de former deux anneaux rotatifs de fluides secondaires tournant autour dudit axe de symétrie cylindrique, le long desdites parois latérales (3) et (5), à l'intérieur de ladite zone libre, et **en ce que** lesdits dispositifs d'alimentation desdits fluides secondaires permettent d'alimenter lesdits fluides secondaires à une pression suffisante pour faire tourner lesdits anneaux rotatifs de fluides secondaires à une vitesse supérieure à la plus grande vitesse de rotation des fluides tournant dans la chambre cyclonique, lorsque celle-ci est en fonctionnement sans le dispositif d'injection de fluide secondaire selon l'invention.

2. Dispositif suivant la revendication 1, **caractérisé en ce que** la dite chambre cyclonique est d'une largeur inférieure à son diamètre et **en ce que** la distance entre les ouvertures (72) d'alimentation de fluide (59) au travers de ladite paroi périphérique est inférieure à son rayon moyen.

3. Dispositif suivant la revendication 1 ou 2, **caractérisé en ce qu'**il comprend au moins un dit tube central d'évacuation des fluides de chaque côté latéral de la chambre cyclonique.

4. Dispositif suivant la revendications 3, **caractérisé en ce qu'**il comprend au moins une paroi de séparation (53), divisant la chambre cyclonique en deux sections transversales, A et B, ladite paroi de séparation ayant au moins un passage (39) permettant le passage de particules solides entraînées par les fluides le long de ladite paroi périphérique (4) d'une dite section transversale à l'autre, et **en ce qu'**il comprend un dispositif d'injection de fluides secondaires (17.2) et (17.3) le long des deux côtés de ladite paroi de séparation, dans une direction principalement tangentielle, dans lesdites zones libres desdites sections transversales.

5. Dispositif suivant l'une des revendications de 1 à 4, **caractérisé en ce que** au moins un dit anneau rotatif desdits fluides secondaires se forme derrière une paroi de guidage (38) avant d'entrer en contact avec les flux de fluides tournant dans ladite zone libre de la chambre cyclonique.

6. Dispositif suivant l'une des revendications de 1 à 5, **caractérisé en ce que** au moins un dispositif d'injection de fluide secondaire peut alimenter au moins 8% du débit de fluides traversant la chambre cyclonique et comprend au moins 8 dites ouvertures d'injection réparties dans ladite chambre d'alimentation centrale.

7. Dispositif suivant l'une des revendications de 1 à 6, **caractérisé en ce qu'**au moins un dit tube central (7) se termine par un conduit à symétrie cylindrique (21), délimité d'un côté par la surface évasée (20) de l'extrémité dudit tube central (7) et de l'autre côté par la surface bombée (26) d'un disque latéral (22) fermant ledit tube central (7) ; ledit conduit à symétrie cylindrique étant fermé par une paroi circulaire (23) de largeur intérieure (49) inférieure au rayon de l'entrée dudit tube central (7) et ladite paroi circulaire (23) ayant au moins 2 ouvertures de sorties tangentielles (24) permettant l'évacuation tangentielle des fluides déviés (28) par ledit conduit à symétrie cylindrique.

8. Dispositif suivant l'une des revendications de 1 à 7, **caractérisé en ce qu'**un dit dispositif d'alimentation de fluides secondaires (17) comprend deux dites chambres contiguës d'alimentation centrale (15.1) et (14), dont l'une permet d'alimenter un gaz secondaire et l'autre un liquide secondaire.

9. Dispositif suivant l'une des revendications de 1 à 8, **caractérisé en ce qu'**il comprend au moins un tube concentrique (80) à un dit tube central (7), pénétrant dans ladite chambre cyclonique et permettant de pulvériser des gouttelettes d'un liquide dans ladite chambre cyclonique.

10. Dispositif suivant l'une des revendications de 1 à 9, **caractérisé en ce que** le dispositif d'évacuation des particules solides comprend au moins une ouverture de sortie (66) dans la paroi périphérique (4) de la chambre cyclonique, entourée d'une jambe de décantation (12) permettant l'accumulation et l'évacuation des particules solides (63) sous l'effet de leur inertie et de la force de la pesanteur lorsque le dispositif fonctionne.

11. Utilisation du dispositif suivant l'une des revendications de 1 à 10 par un procédé de transformation de fluides et de particules solides tournant dans une chambre cyclonique, comprenant la séparation desdites particules solides et desdits fluides, **caractérisé en ce que** lesdits flux de fluides sortant en entraînant des particules solides le long desdites parois latérales sont écartés desdites parois latérales et leur vitesse de rotation est accélérée par l'injection desdits fluides secondaires le long desdites parois latérales.

12. Procédé de transformation de particules solides et de fluides tournant dans une chambre cyclonique comprenant un dispositif d'injection de fluides secondaires suivant l'une des revendications de 1 à 10, **caractérisé en ce que** au moins un desdits fluides secondaires réagit avec au moins un desdits flux de fluides sortant le long d'une dite paroi latérale ou de séparation.

13. Procédé de transformation de particules solides et de fluides tournant dans une chambre cyclonique, suivant la revendication 12, **caractérisé en ce qu'**il comprend au moins deux dites chambres contiguës d'alimentation centrale, l'une injectant un liquide secondaire et l'autre un gaz secondaire, ledit liquide secondaire réagissant avec les particules solides entraînées par undit flux de fluides sortant le long d'une dite paroi latérale ou de séparation.

14. Procédé de combustion de matières carbonées dans une chambre cyclonique suivant la revendication 12 ou 13, **caractérisé en ce que** les fluides alimentés tangentiellement au travers de la paroi circulaire (4) ne contiennent pas suffisamment d'oxygène pour obtenir une combustion complète desdites matières carbonées et **en ce qu'**au moins un dit fluide secondaire contient l'oxygène nécessaire pour achever l'oxydation desdits flux de fluides sortant le long d'une paroi latérale.

15. Procédé de transformation de fluides et de particules solides comprenant la transformation alternée desdites particules solides dans une chambre cyclonique divisée en deux sections transversales suivant une des revendications 12 à 14, **caractérisé en ce qu'**il comprend une réaction endothermique dans une section et une réaction exothermique dans l'autre section et **en ce qu'**au moins un desdits fluides secondaires réagit avec au moins un desdits flux de fluides sortants.

## Patentansprüche

1. Vorrichtung zur Einspritzung von Sekundärfluiden ins Innere des freien Bereichs einer rotierenden Wirbelschicht in einer ortsfesten Zyklonkammer, welche umfasst: eine Umfangswand (4), die von zwei Seitenwänden (3) und (5) umgeben ist; eine Vorrichtung zur Zuführung von Fluid (57) durch Öffnungen (72) hindurch, die entlang der Umfangswand verteilt sind, in einer hauptsächlich tangentialen Richtung (73); eine Vorrichtung zur Zuführung und Abführung von festen Partikeln; wenigstens ein zentrales Rohr (7) zur Ableitung der Fluide, die in der Zyklonkammer rotieren; **dadurch gekennzeichnet, dass** sie umfasst:
- eine Vorrichtung zur Zuführung von Sekundärfluiden (17) und (17.1) durch zentrale Zuführungskammern (15) und (15.1) hindurch, die entlang jeder der Seitenwände (3) und (5) angeordnet sind und mit Einspritzöffnungen (18) und (18.1) versehen sind, welche um die Zylindersymmetrieachse herum verteilt sind und ermöglichen, die Sekundärfluide in einer hauptsächlich tangentialen Richtung (19) und (19.1) einzuspritzen, um zwei rotierende Ringe von Sekundärfluiden, die sich um die Zylindersymmetrieachse drehen, entlang der Seitenwände (3) und (5) im Inneren des freien Bereichs zu bilden, und dadurch, dass die Vorrichtungen zur Zuführung der Sekundärfluide ermöglichen, die Sekundärfluide mit einem Druck zuzuführen, der ausreichend ist, um zu bewirken, dass sich die rotierenden Ringe von Sekundärfluiden mit einer Geschwindigkeit drehen, die höher als die größte Rotationsgeschwindigkeit der Fluide ist, die in die Zyklonkammer rotieren, wenn diese ohne die erfindungsgemäße Vorrichtung zur Einspritzung von Sekundärfluid in Betrieb ist.

2. Vorrichtung nach Anspruch 1, **dadurch gekennzeichnet, dass** die Zyklonkammer eine Breite aufweist, die kleiner als ihr Durchmesser ist, und dadurch, dass der Abstand zwischen den Öffnungen (72) zur Zuführung von Fluid (59) durch die Umfangswand hindurch kleiner als deren mittlerer Radius ist.

3. Vorrichtung nach Anspruch 1 oder 2, **dadurch gekennzeichnet, dass** sie wenigstens ein sogenanntes zentrales Rohr zur Ableitung der Fluide auf jeder lateralen Seite der Zyklonkammer umfasst.

4. Vorrichtung nach Anspruch 3, **dadurch gekennzeichnet, dass** sie wenigstens eine Trennwand (53) umfasst, welche die Zyklonkammer in zwei Querabschnitte A und B aufteilt, wobei die Trennwand wenigstens einen Durchlass (39) aufweist, der die Hindurchbewegung von festen Partikeln, die von den Fluiden mitgenommen werden, entlang der Umfangswand (4) von einem solchen Querabschnitt zum anderen ermöglicht, und dadurch, dass sie eine Vorrichtung zur Einspritzung von Sekundärfluiden (17.2) und (17.3) entlang der zwei Seiten der Trennwand in einer hauptsächlich tangentialen Richtung in die freien Bereiche der Querabschnitte umfasst.

5. Vorrichtung nach einem der Ansprüche 1 bis 4, **dadurch gekennzeichnet, dass** sich wenigstens ein rotierender Ring der Sekundärfluide hinter einer Führungswand (38) bildet, bevor er mit den Strömen von Fluiden in Kontakt kommt, die in dem freien Bereich der Zyklonkammer rotieren.

6. Vorrichtung nach einem der Ansprüche 1 bis 5, **dadurch gekennzeichnet, dass** wenigstens eine Vorrichtung zur Einspritzung von Sekundärfluid mindestens 8 % der Durchflussmenge von Fluiden zuführen kann, welche die Zyklonkammer durchströmen, und mindestens 8 solche Einspritzöffnungen umfasst, die in der zentralen Zuführungskammer verteilt sind.

7. Vorrichtung nach einem der Ansprüche 1 bis 6, **dadurch gekennzeichnet, dass** wenigstens ein besagtes zentrales Rohr (7) in einem zylindersymmetrischen Kanal (21) endet, der auf einer Seite von der aufgeweiteten Fläche (20) des Endes des zentralen Rohres (7) und auf der anderen Seite von der gewölbten Fläche (26) einer seitlichen Scheibe (22), die das zentrale Rohr (7) verschließt, begrenzt wird; wobei der zylindersymmetrische Kanal von einer kreisförmigen Wand (23) mit einer inneren Breite (49), die kleiner als der Radius des Eingangs des zentralen Rohres (7) ist, verschlossen wird und die kreisförmige Wand (23) wenigstens 2 tangentiale Austrittsöffnungen (24) aufweist, welche die tangentiale Ableitung der durch den zylindersymmetrischen Kanal abgelenkten Fluide (28) ermöglichen.

8. Vorrichtung nach einem der Ansprüche 1 bis 7, **dadurch gekennzeichnet, dass** eine besagte Vorrichtung zur Zuführung von Sekundärfluiden (17) zwei aneinander angrenzende zentrale Zuführungskammern (15.1) und (14) umfasst, von denen eine ermöglicht, ein Sekundärgas, und die andere, eine Sekundärflüssigkeit zuzuführen.

9. Vorrichtung nach einem der Ansprüche 1 bis 8, **dadurch gekennzeichnet, dass** sie wenigstens ein mit einem besagten zentralen Rohr (7) konzentrisches Rohr (80) umfasst, das in die Zyklonkammer hineinführt und ermöglicht, Tröpfchen einer Flüssigkeit in der Zyklonkammer zu zerstäuben.

10. Vorrichtung nach einem der Ansprüche 1 bis 9, **dadurch gekennzeichnet, dass** die Vorrichtung zur Abführung der festen Partikel wenigstens eine Austrittsöffnung (66) in der Umfangswand (4) der Zyklonkammer umfasst, die von einem Dekantierungselement (12) umgeben ist, das die Ansammlung und die Abführung der festen Partikel (63) unter der Wirkung ihrer Trägheit und der Schwerkraft ermöglicht, wenn die Vorrichtung in Betrieb ist.

11. Verwendung einer Vorrichtung nach einem der Ansprüche 1 bis 10 bei einem Verfahren zur Transformation von Fluiden und von festen Partikeln, die in einer Zyklonkammer rotieren, welches die Trennung der festen Partikel und der Fluide umfasst, **dadurch gekennzeichnet, dass** die Ströme von Fluiden, die entlang der Seitenwände austreten und dabei feste Partikel mitführen, durch Einspritzung der Sekundärfluide entlang der Seitenwände auf einen Abstand von den Seitenwänden gebracht werden und ihre Rotationsgeschwindigkeit beschleunigt wird.

12. Verfahren zur Transformation von festen Partikeln und von Fluiden, die in einer Zyklonkammer rotieren, welche eine Vorrichtung zur Einspritzung von Sekundärfluiden nach einem der Ansprüche 1 bis 10 umfasst, **dadurch gekennzeichnet, dass** wenigstens eines der Sekundärfluide mit wenigstens einem der Fluidströme reagiert, die entlang einer der besagten Seiten- oder Trennwände austreten.

13. Verfahren zur Transformation von festen Partikeln und von Fluiden, die in einer Zyklonkammer rotieren, nach Anspruch 12, **dadurch gekennzeichnet, dass** es wenigstens zwei besagte aneinander angrenzende zentrale Zuführungskammern umfasst, von denen die eine eine Sekundärflüssigkeit und die andere ein Sekundärgas einspritzt, wobei die Sekundärflüssigkeit mit den festen Partikeln reagiert, die von einem der Fluidströme mitgeführt werden, die entlang einer der besagten Seiten- oder Trennwände austreten.

14. Verfahren zur Verbrennung von kohlenstoffhaltigen Materialien in einer Zyklonkammer nach Anspruch 12 oder 13, **dadurch gekennzeichnet, dass** die Fluide, die tangential durch die kreisförmige Wand (4) hindurch zugeführt werden, nicht genügend Sauerstoff enthalten, um eine vollständige Verbrennung der kohlenstoffhaltigen Materialien zu erzielen, und dadurch, dass wenigstens ein besagtes Sekundärfluid den erforderlichen Sauerstoff enthält, um die Oxidation der entlang einer Seitenwand austretenden Fluidströme zu vollenden.

15. Verfahren zur Transformation von Fluiden und von festen Partikeln, welches die abwechselnde Transformation der festen Partikel in einer Zyklonkammer, die in zwei Querabschnitte aufgeteilt ist, nach einem der Ansprüche 12 bis 14 umfasst, **dadurch gekennzeichnet, dass** es eine endotherme Reaktion in einem Abschnitt und eine exotherme Reaktion im anderen Abschnitt umfasst, und dadurch, dass wenigstens eines der Sekundärfluide mit wenigstens einem der austretenden Fluidströme reagiert.

## Claims

1. Device for injecting secondary fluids into the free area of a rotating fluidized bed in a fixed cyclone chamber, comprising a peripheral wall (4) surrounded by two side walls (3) and (5); a device for supplying fluid (57) through openings (72) distributed along said peripheral wall, in a mainly tangential direction (73); a device for supplying and discharging solid particles; and at least one central tube (7) for discharging the fluids revolving in said cyclone chamber, **characterized in that** it comprises:
- a device for supplying secondary fluids (17) and (17.1) through central supply chambers (15) and (15.1) located along each of said side walls (3) and (5), having injection openings (18) and (18.1) distributed around the axis of cylindrical symmetry, enabling said secondary fluids to be injected in a mainly tangential direction (19) and (19.1), in order to form two rotating rings of secondary fluids revolving around said axis of cylindrical symmetry, along said side walls (3) and (5), within said free area, and **in that** said devices for supplying said secondary fluids enable said secondary fluids to be supplied at a sufficient pressure to make said rotating rings of secondary fluids revolve at a velocity which is greater than the highest rotation velocity of said fluids revolving in cyclone chamber when the latter is in operation without the device for injecting secondary fluid according to the invention.

2. Device according to Claim 1, **characterized in that** said cyclone chamber has a width smaller than its diameter, and **in that** the distance between the openings (72) for supplying fluid (59) through said peripheral wall is smaller than its mean radius.

3. Device according to Claim 1 or 2, **characterized in that** it comprises at least one said central tube for discharging the fluids from each lateral side of the cyclone chamber.

4. Device according to Claim 3, **characterized in that** it comprises at least one separating wall (53), dividing the cyclone chamber into two transverse sections A and B, said separating wall having at least one passage (39) allowing the passage of solid particles entrained by the fluids along said peripheral wall (4) from one said transverse section to the other, and **in that** it comprises a device for injecting secondary fluids (17.2) and (17.3) along the two sides of said separating wall, in a mainly tangential direction, into said free areas of said transverse sections.

5. Device according to any of Claims 1 to 4, **characterized in that** at least one said rotating ring of said secondary fluids is formed behind a guide wall (38) before coming into contact with the fluid flows revolving in said free area of the cyclone chamber.

6. Device according to any of Claims 1 to 5, **characterized in that** at least one device for injecting secondary fluid may supply at least 8% of the flow rate of fluids passing through the cyclone chamber, and comprises at least eight said injection openings distributed in said central supply chamber.

7. Device according to any of Claims 1 to 6, **characterized in that** at least one said central tube (7) terminates in a cylindrically symmetric conduit (21), delimited on one side by the flared surface (20) of the end of said central tube (7), and on the other side by the curved surface (26) of a lateral disk (22) closing said central tube (7); said cylindrically symmetric conduit being closed by a circular wall (23) whose inside width (49) is less than the radius of the inlet of said central tube (7), said circular wall (23) having at least two tangential outlet openings (24) for the tangential discharge of the fluids (28) deflected by said cylindrically symmetric conduit.

8. Device according to any of Claims 1 to 7, **characterized in that** a said device for supplying secondary fluids (17) comprises two said contiguous central supply chambers (15.1) and (14), of which one may be used to supply a secondary gas and the other may be used to supply a secondary liquid.

9. Device according to any of Claims 1 to 8, **characterized in that** it comprises at least one tube (80) concentric with a said central tube (7), penetrating into the cyclone chamber and enabling droplets of a liquid to be atomized in said cyclone chamber.

10. Device according to any of Claims 1 to 9, **characterized in that** the device for discharging the solid particles comprises at least one outlet opening (66) in the peripheral wall (4) of the cyclone chamber, surrounded by a decantation tube (12) for the accumulation and discharge of the solid particles (63) under the effect of their inertia and the force of gravity when the device is operating.

11. Use of the device according to any of Claims 1 to 10 by a method for processing fluids and solid particles revolving in a cyclone chamber, comprising the separation of said solid particles and said fluids, **characterized in that** said fluid flows exiting while entraining solid particles along said side walls are separated from said side walls, and their rotation velocity is accelerated by the injection of said secondary fluids along said side walls.

12. Method for processing solid particles and fluids revolving in a cyclone chamber comprising a device for injecting secondary fluid according to any of Claims 1 to 10, **characterized in that** at least one of said secondary fluids reacts with at least one of said fluid flows exiting along a said side wall or separating wall.

13. Method for processing solid particles and fluids revolving in a cyclone chamber according to Claim 12, **characterized in that** it comprises at least two said contiguous central supply chambers, one injecting a secondary liquid and the other injecting a secondary gas, said secondary liquid reacting with the solid particles entrained by a said fluid flow exiting along a said side wall or separating wall.

14. Method for the combustion of carbonaceous matter revolving in a cyclone chamber according to Claim 12 or 13, **characterized in that** the fluids injected tangentially through the circular wall (4) do not contain enough oxygen to provide complete combustion of said carbonaceous matter, and **in that** at least one said secondary fluid supplied by a device according to the invention contains the necessary oxygen to complete the oxidation of said fluid flows exiting along a side wall.

15. Method for processing fluids and solid particles, comprising the alternating processing of said solid particles in a cyclone chamber divided into two transverse sections according to any of Claims 12 to 14, **characterized in that** it comprises an endothermic reaction in one section and an exothermic reaction in the other section, and **in that** at least one of said secondary fluids reacts with at least one of said exiting fluid flows.
